# EUROPEAN PATENT SPECIFICATION

(11) **EP 4 312 861 B1**
(45) Date of publication and mention of the grant of the patent: **18.02.2026**
(21) Application number: 22715746.8
(22) Date of filing: 01.04.2022
(51) Int. Cl.: A61B 46/20

(54) **SURGICAL INCISE DRAPE**
CHIRURGISCHES INZISIONSTUCH
CHAMP OPÉRATOIRE D'INCISION CHIRURGICALE

(30) Priority: 02.04.2021 US 202163169990 P
(43) Date of publication of application: 07.02.2024
(73) Proprietor: Solventum Intellectual Properties Company, Maplewood, MN 55144 (US)
(72) Inventor: BENNAARS-EIDEN, Assumpta A.G., Saint Paul, Minnesota 55133-3427 (US); STANKARD, Philip N., Saint Paul, Minnesota 55133-3427 (US); SCHNEIDER, Joanna Esther Z., Saint Paul, Minnesota 55133-3427 (US); KOCH, Diane L., Saint Paul, Minnesota 55133-3427 (US); SPERLING, John W., Rochester, Minnesota 55902 (US); JACOBSON, Richard L., Saint Paul, Minnesota 55133-3427 (US)
(74) Representative: Vossius & Partner Patentanwälte Rechtsanwälte mbB
(86) International application number: PCT/IB2022/053083
(87) International publication number: WO 2022/208476

(56) References cited:
- WO-A1-2021/044309
- US-A- 4 643 181
- US-A- 5 986 162
- US-A1- 2013 125 901
- US-A1- 2016 007 980

## Description

### BACKGROUND

Surgical-site infections often lead to prolonged hospitalizations and are heavily associated with high morbidity and mortality. A patient's risk for surgical-site infection is largely proportional to the residual bacteria at, or in proximity to, the surgical site. The steady increase in antibiotic resistance makes pre-operative efforts to reduce bacteria density at surgical sites crucial.

Disinfecting procedures reduce the risk of infection, as does covering the surgical site with a surgical incise drape having an antimicrobial-impregnated adhesive. However, contoured areas are difficult to adequately drape since creasing or wrinkling of the surgical incise drape creates a gap between the skin and the drape. Gaps prevent thorough and continued disinfection and may allow for further bacterial introduction.

Shoulder operations are particularly susceptible to infection due to the difficulty in draping a patient's arm. Standard surgical incise drapes are square or rectangular in shape and often wrinkle, crease, or otherwise fold onto themselves when trying to drape portions of the arm. US 4 643 181 A describes surgical dressings and incise drapes which have an adhesive surface to secure the dressing or drape to a patient and in which the adhesive contains a salt of polyhexamethylene biguanide as a broad spectrum antimicrobial agent.

What is needed is a surgical incise drape or set of or surgical incise drapes in a shape that is capable of covering the contours of the arm.

### SUMMARY

The surgical incise drape according to the invention is defined in claim 1. Embodiments are defined in the dependent claims. The method of preparing a surgical incise drape according to the invention is defined in claim 14.

In one embodiment, a surgical incise drape is described. The surgical incise drape includes a flexible substrate having a trapezoidal configuration including four vertices A, B, C, and D; and an antimicrobial adhesive composition. The flexible substrate includes a first base *(AB)* defined by a first base length (*AB*_{L}) measured between vertices A and B; a second base *(CD)* defined by a second base length (*CD*_{L}) measured between vertices C and D, wherein *AB*_{L} *> CD*_{L}; a first side *(AC)* defined by a first side length (*AC*_{L}) measured between vertices A and C; and a second side *(BD)* defined by a second side length (*BD*_{L}) measured between vertices B and D. The antimicrobial adhesive composition includes an antimicrobial agent and a pressure-sensitive adhesive.

In one embodiment, a surgical incise drape is described. The surgical incise drape includes a flexible substrate divided into three separate subsections, wherein the three separate sections together form a trapezoidal configuration characterized by four vertices A, B, C, and D; and an antimicrobial adhesive composition. The flexible substrate includes a first base (*AB*) defined by a first base length (*AB*_{L}) measured between vertices A and B; a second base (*CD*) defined by a second base length (*CD*_{L}) measured between vertices C and D, wherein *AB*_{L} *> CD*_{L}; a first side (*AC*) defined by a first side length (*AC*_{L}) measured between vertices A and C; and a second side (*BD*) defined by a second side length (*BD*_{L}) measured between vertices B and D. The antimicrobial adhesive composition includes an antimicrobial agent and a pressure-sensitive adhesive.

A method of preparing a surgical incise drape is described. The method includes providing a flexible substrate and an antimicrobial adhesive composition; and disposing the antimicrobial adhesive composition onto the flexible substrate.

A method for preparing a shoulder for surgery is described. The method includes providing a surgical incise drape described herein; and contacting the surgical incise drape to a skin surface of a subject.

A kit is described. The kit includes one or more surgical incise drapes described herein; and a set of instructions directed a user to perform method steps for preparing a shoulder for surgery.

### BRIEF DESCRIPTION OF DRAWINGS

**FIG. 1A** illustrates a surgical incise drape of the present disclosure.
**FIG. 1B** illustrates a surgical incise drape of the present disclosure having contouring.
**FIG. 1C** illustrates a surgical incise drape of the present disclosure having a delivery system.
**FIG. 1D** illustrates a surgical incise drape of the present disclosure having a delivery system with handling tabs.
**FIG. 1E** illustrates a surgical incise drape of the present disclosure having a delivery system with handling tabs.
**FIG. 1F** illustrates surgical incise drape of the present disclosure having contouring and a delivery system.
**FIG. 2A** illustrates a surgical incise drape of the present disclosure.
**FIG. 2B** illustrates a surgical incise drape of the present disclosure having contouring.
**FIG. 2C** illustrates a surgical incise drape of the present disclosure having a delivery system.
**FIG. 2D** illustrates a surgical incise drape of the present disclosure having subsection contouring.
**FIG. 2E** illustrates a separated three-piece surgical incise drape of the present disclosure having subsection contouring.
**FIG. 3** illustrates a surgical incise drape of the present disclosure overlaying a subject's arm.
**FIG. 4A** illustrates a cross-sectional view of a surgical incise drape of the present disclosure.
**FIG. 4B** illustrates a cross-sectional view of a surgical incise drape of the present disclosure.
**FIG. 4C** illustrates a cross-sectional view of a surgical incise drape of the present disclosure.
**FIG. 5** is a flow chart of a method for preparing a surgical incise drape of the present disclosure.
**FIG. 6** is a flow chart of a method for preparing a subject's shoulder for surgery.
**FIG. 7** illustrates a kit including a surgical incise drape of the present disclosure.

### DETAILED DESCRIPTION

*Proprionibacterium acnes* commonly colonizes the skin, among other infectious organisms, and is responsible for more than half of the post-operative infections involving the shoulder. Several technological advances have limited surgical site infections, most notably the use of surgical incise drapes, however it is challenging to drape contoured areas of the body (e.g., shoulder), which is largely due to wrinkling of the drape. When a surgical incise drape wrinkles, areas beneath the wrinkles are not adequately disinfected, thereby leading to an increase in chance for infection.

The surgical incise drapes of the present disclosure directly address the challenges of draping the shoulder and surrounding areas. The trapezoidal configuration serves to limit or eliminate wrinkling of the drape upon application to the skin surface. Additional features of the drape construction, e.g., contouring, delivery systems, and drape sectioning, further assist in application of the drape and assure greater skin contact. Delivery systems provide structural reinforcement which can prevent drapes from creasing or otherwise folding onto themselves during application. Moreover, the delivery systems of the present disclosure may be removed in portions, thus allowing piecewise application of the drape meanwhile maintaining reinforcement for the remainder of the drape. In addition to greater application control, three-part drape sectioning (i.e., drape seal, incise seal, and stockinette seal) would allow for surgeons to modify the drape in view of the type of surgery. For example, anthroscopic surgery may only require a drape seal and a stockinette seal as compared to open surgery requiring the full drape.

### Definitions

As used herein, "about" means ± 10 percent of a given value. For example, about 10 means 9 to 11.

As used herein, "contouring" refers to a structural impression that deviates from the overall trapezoidal configuration. All non-straight features are considered contouring. Contouring allows for the surgical incise drapes of the present disclosure to be more readily placed around body structures. "Concave contouring" refers to surface area removed from the trapezoid configuration defined by vertices A, B, C, and D, or surface are removed from subsectional areas (i.e., subsection seals). "Convex contouring refers to surface area added to the overall trapezoid configuration, i.e., surface area extending beyond the trapezoid configuration defined by vertices A, B, C, and D, or surface area added to subsectional areas. Contouring may prevent wrinkling.

As used herein, "disinfecting" refers to a reduction in the number of active microorganisms present on a surface being disinfected. Disinfecting may kill or prevent microorganisms from growing or proliferating.

As used herein, "plasticizer" refers to a substance or combination of substances that lowers the glass transition temperature of another substance (e.g., a pressure-sensitive adhesive). Plasticizers effectively soften, increase flexibility, increase plasticity, decrease viscosity, and/or decrease friction of a substance to which it is added. The plasticizers described herein are relatively low molecular weight polymers that increase the spacing between chains of a larger molecular weight polymer (i.e., PSA).

As used herein, "polymer" refers to a substance having one or more repeating monomer units. The chemical identities of the polymeric substances herein are at times described in terms of the monomers to which the polymer is derived. A skilled artisan would readily understand the reactivity profile of the recited monomers and how the monomers could synthetically be joined to form the polymer.

As used herein, "pressure-sensitive adhesive" refers to a non-reactive, self-stick adhesive that forms a bond when pressure is applied. No solvent, water, or heat is required to activate a pressure-sensitive adhesive.

When referring to "solubility," or "to solubilize" it should be understood that the solubility of a component A in a component B refers to conditions in which only component A and component B are present, e.g., no added salts, compounds, or the like. Furthermore, any solubility values provided herein are with regard to a temperature range of about 20 °C to about 23 °C at atmospheric pressure (i.e., 760 mm/Hg).

As used herein, "trapezoid" refers to a shape consisting of four vertices joined by straight lines. Two lines join to form a vertex at an angle. No more than two angles have the same value.

As used herein, "vertices A and B" are determined to be the points defining the greatest width distance (i.e., *AB_{L}*) within the flexible substrate. "Vertices C and D" are determined to be the points defining the least width distance (i.e., *CD_{L}*) within the flexible substrate. Any surface area extending beyond the vertices is considered contouring.

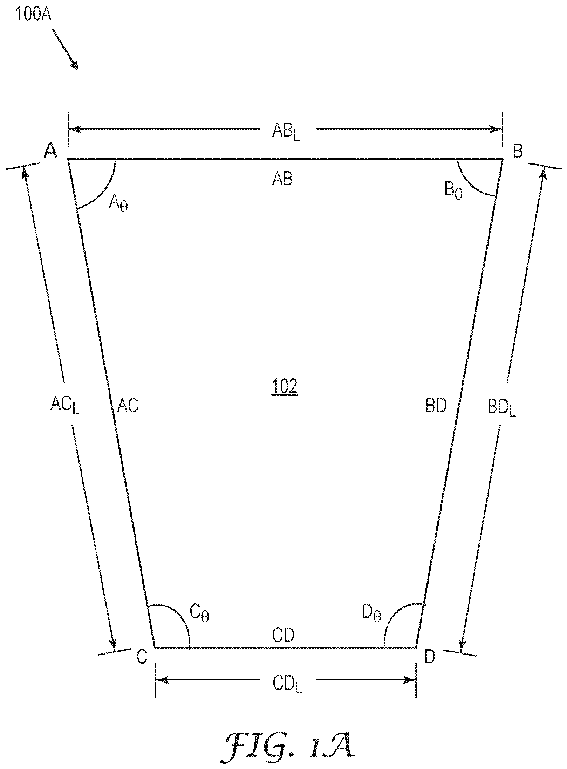

**FIG. 1A** depicts a one-piece trapezoidal surgical incise drape **100A** of the present disclosure. Surgical incise drape **100** includes a flexible substrate **102** having a first base ***AB*** defined by a first base length ***AB*_{L}** as measured between vertices **A** and **B,** a second base ***CD*** defined by a second base length ***CD*_{L}** as measured between vertices **C** and **D,** a first side ***AC*** defined by a first side length ***AC*_{L}** as measured between vertices **A** and **C,** and a second side ***BD*** defined by a second side length ***BD*_{L}** as measured between vertices **B** and **D.** As depicted, first length ***AB*_{L}** is greater than second length ***CD*_{L}*.*** First base **AB** and first side **AC** join at vertex A at an angle **A_{θ}**. First base **AB** and second side **BD** join at vertex B at an angle **B_{θ}**. Second base **CD** and first side **AC** join at vertex ***C*** at an angle ***C*_{θ}**. Second base **CD** and second side **BD** join at vertex **D** at an angle **D_{θ}**. Surgical incise drape **100A** further includes an antimicrobial adhesive composition (not shown) having an antimicrobial agent and a pressure-sensitive adhesive contacting a major surface of the flexible substrate **202.**

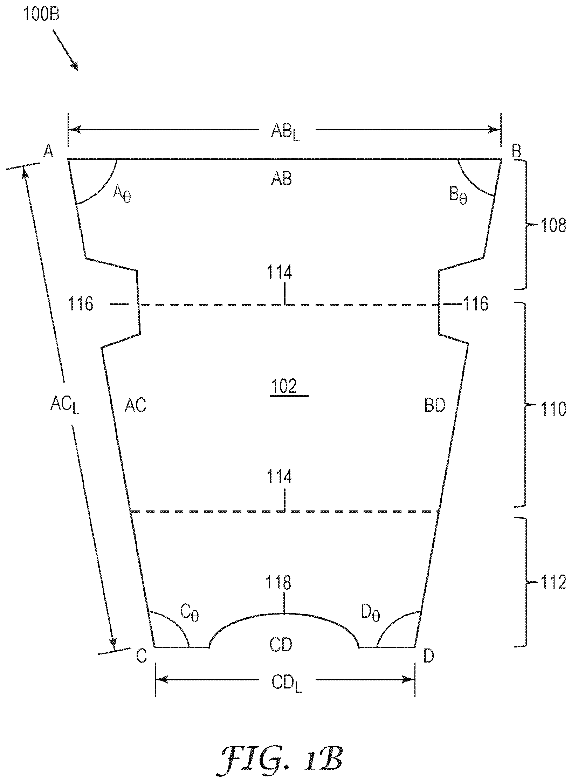

**FIG. 1B** depicts a one-piece trapezoidal surgical incise drape **100B** having all the features of surgical incise drape **100A.** All surgical incise drapes of the present disclosure include areas configured to drape sections of a subject's arm in preparation for surgery. These areas are referred to flexible substrate subsections. As depicted, the flexible substrate subsections include: a drape seal **108,** an incise site seal **110,** and a stockinette seal **112.** Drape seal **108** configured to overlay the skin surfaces above the proximal end of a humerus, the distal end of a clavicle, and at least a portion of the neck that is typically covered in nonwoven drape material. Drape seal **108** could adhere to nonwoven drape material under current surgical practices. Incise site seal **110** is configured to overlay the shoulder, bicep, tricep, and wrap to cover the under-armpit area. Stockinette seal **112** is configured to overlay and at least partially encircle the forearm. As depicted, flexible substrate **102** includes printed markings **114.** Flexible substrate **102** is further shown to include side contouring **116** and base contouring **118.** Contouring may assist in the application of surgical incise drape **100B** and prevent wrinkling of flexible substrate **102.**

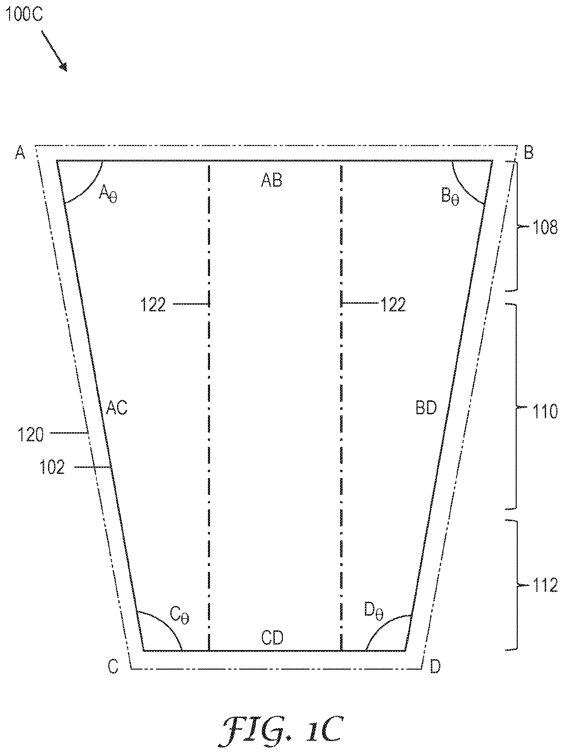

**FIG. 1C** depicts a one-piece trapezoidal surgical incise drape **100C** having all the features of surgical incise drape **100A.** Surgical incise drape **100C** further includes a delivery system **120.** As depicted, delivery system **120** is in the form of a surface liner covering the entire area of flexible substrate **102.** In this particular illustration, delivery system **120** includes two cuts **122** that separate the delivery system into three liner sections, in which the liner sections may be removed independently. A sectioned delivery system may allow for easier application of surgical incise drape **100C** and prevent wrinkling.

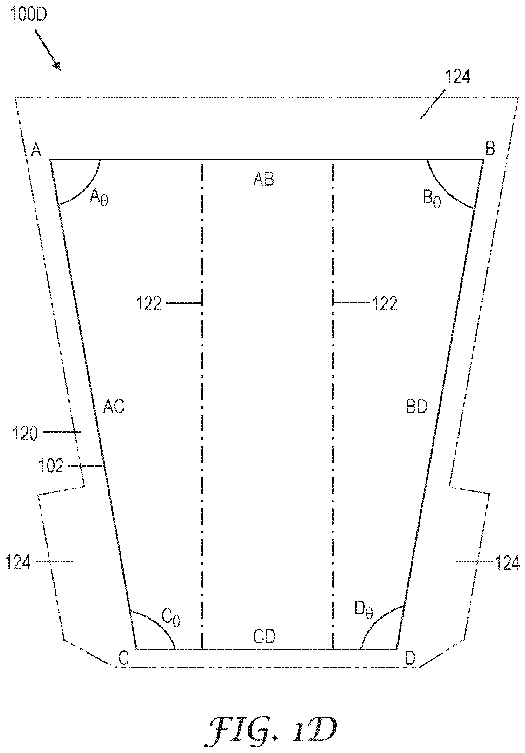

**FIG. 1D** depicts a one-piece trapezoidal surgical incise drape **100D** having all the features of surgical incise drape **100C.** Delivery system **120,** as shown, includes handling tabs **124.** Handling tabs **124** may further assist in application of surgical incise drape **100D.**

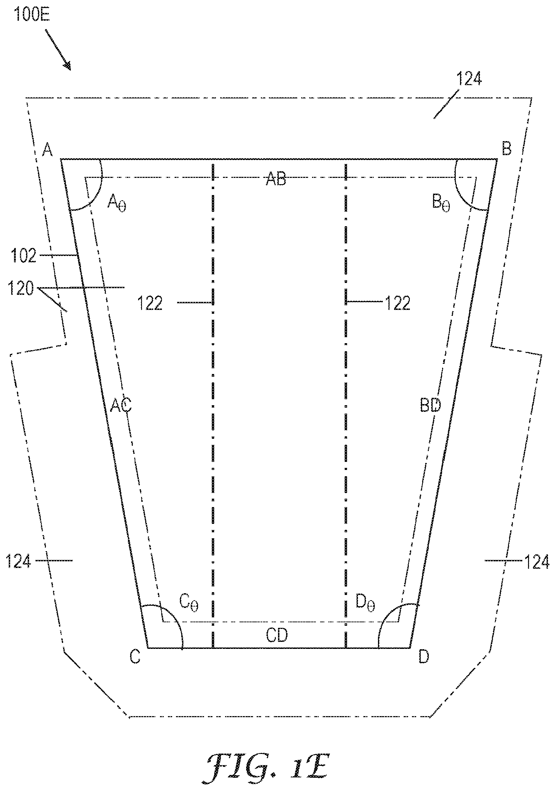

**FIG. 1E** depicts a one-piece trapezoidal surgical incise drape **100E** having all the features of surgical incise drape **100D.** Delivery system **120,** as shown, includes two liners - a surface area liner and a perimeter liner. The perimeter liner is ideally constructed from a material more rigid than a material suitable for a surface liner. The perimeter liner may aid in handling of surgical incise drape **100E,** e.g., preventing the drape from folding onto itself during handling.

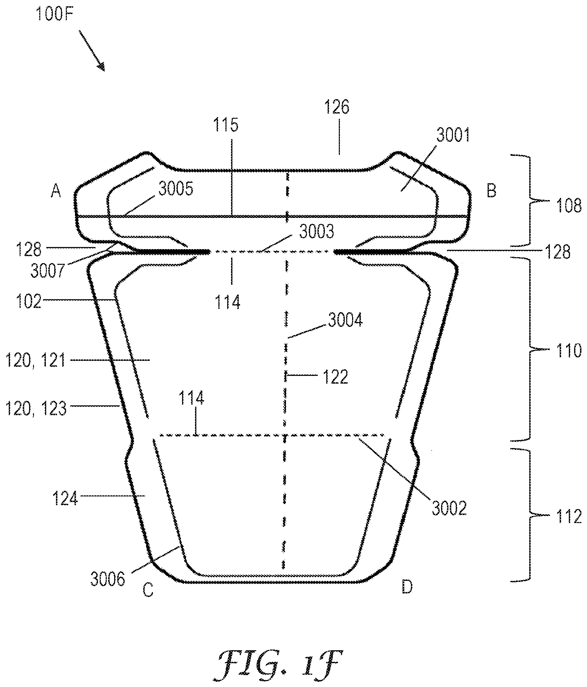

**FIG. 1F** depicts a one-piece trapezoidal surgical incise drape **100F.** Surgical incise drape **100F** is shown to include a flexible substrate **102** having contouring **126** along first base *AB* and contouring **128** along first side *AC* and second side *BD.* Contouring **128** resides between drape seal **108** and surgical incise seal **110.** Surgical incise drape **100F** is further shown to include a delivery system **120** having a surface area liner **121** and a perimeter liner **123** (including handling tabs **124**). Surface liner **121** includes cut lines **114** (printing markings or perforations) in which a user may cut along said lines to form a three-piece drape. Surface liner **121** further includes perforation **122** for sectional removal of the liner, and guide marking **115** for aiding in proper placement of drape seal **108.**

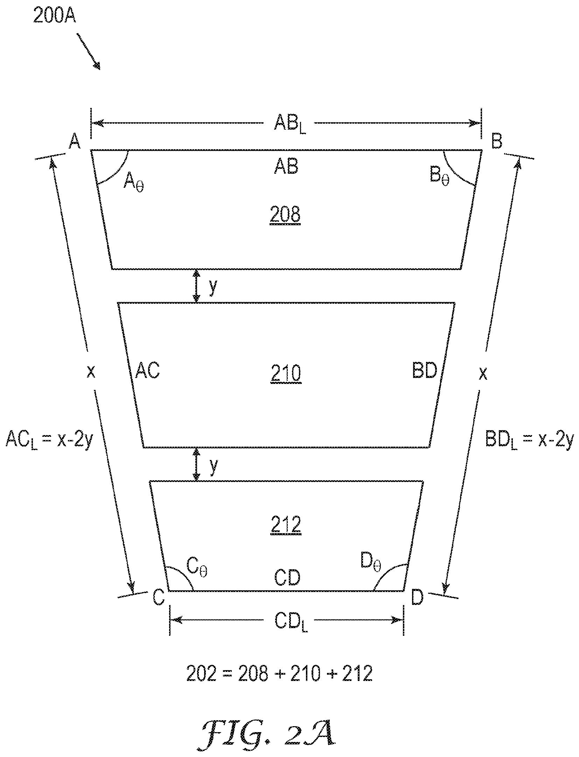

**FIG. 2A** depicts a three-piece trapezoidal surgical incise drape **200A** of the present disclosure. Surgical incise drape **200A** includes all the features of surgical incise drape **100A,** including three flexible substrate subsections **208** (i.e., drape seal), **210** (i.e., incise site seal), and **212** (i.e., stockinette seal) that when nested together form flexible substrate **202** having a trapezoidal configuration.

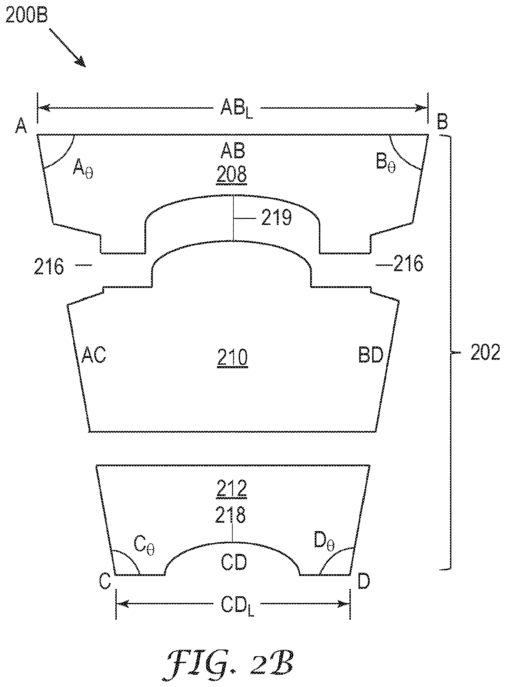

**FIG. 2B** depicts a three-piece trapezoidal surgical incise drape **200B** of the present disclosure having all the features of surgical incise drape **200A.** Flexible substrate **202** is further shown to include side contouring **216,** base contouring **218,** and subsection contouring **219.** Contouring may assist in the application of surgical incise drape **100B** and prevent wrinkling of flexible substrate **102.** For example, subsection contouring, such as **219,** may allow for greater precision in applying subsections **208** and **210** around a shoulder area.

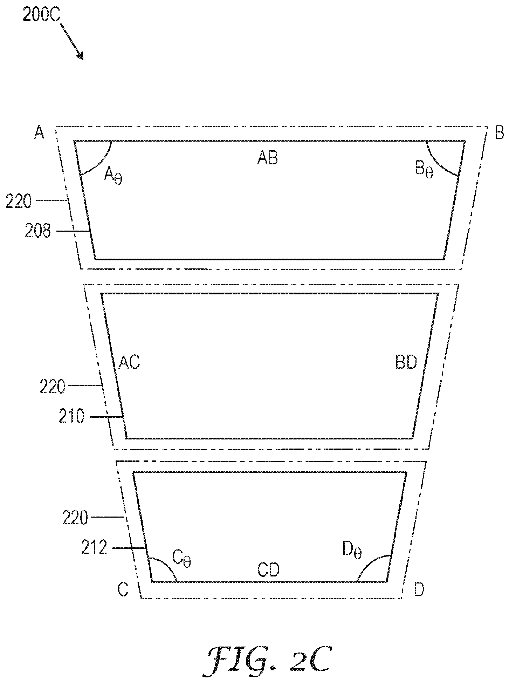

**FIG. 2C** depicts a three-piece trapezoidal surgical incise drape **200C** of the present disclosure having all the features of surgical incise drape **200A.** Surgical incise drape **200C** is shown further including a delivery system **320** (surface liner) on each of the flexible substrate subsections **308, 310,** and **312.** Although not shown, delivery system **320** may further include handling tabs and/or a perimeter liner on one or more of the flexible substrate subsections.

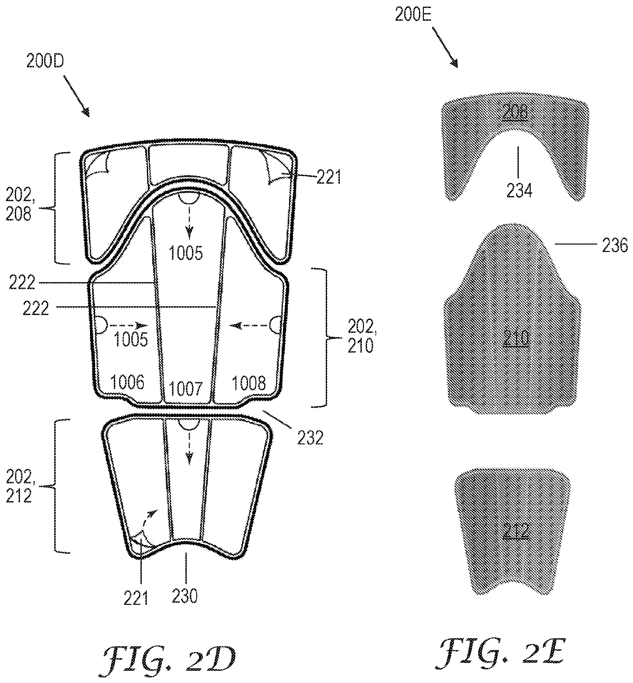

**FIG. 2D** depicts a three-piece trapezoidal surgical incise drape **200D** of the present disclosure. Surgical incise drape **200D** is shown to include a flexible substrate **202** (subsections: drape seal **208,** surgical incise seal **210,** and stockinette seal **212**) having second base *CD* contouring **230** and first side *AC* and second side *BD* contouring **232.** A delivery system (i.e., surface area liner **221**) is shown having two liner perforations **222** for sectioned removal.

**FIG. 2E** depicts three-piece trapezoidal surgical incise drape **200E** wherein flexible substrate subsections **208, 210,** and **212** are separated. As shown drape seal **208** includes concave contouring **234** and surgical incise seal **210** includes convex contouring **236,** wherein contouring **234** and **236** are configured to nest together. This particular contouring pattern may allow for better coverage of the shoulder region.

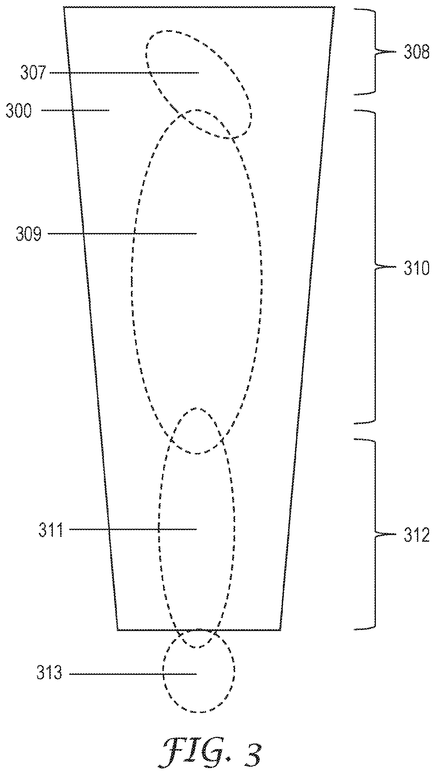

**FIG. 3** depicts a trapezoidal surgical incise drape **300** of the present disclosure overlaying a subject's arm. The subject's arm is divided into four sections: a neck / clavicle region **307,** a shoulder / upper arm region **309,** a forearm region **311,** and a hand region **313.** Surgical incise drape **300** is shown to include three flexible substrate subsections: drape seal **308,** surgical incise seal **310,** and stockinette seal **312.** Although shown as a one-piece construction, one can easily envision the flexible substrate subsections separated into a three-piece constructions and nested in the trapezoidal configuration.

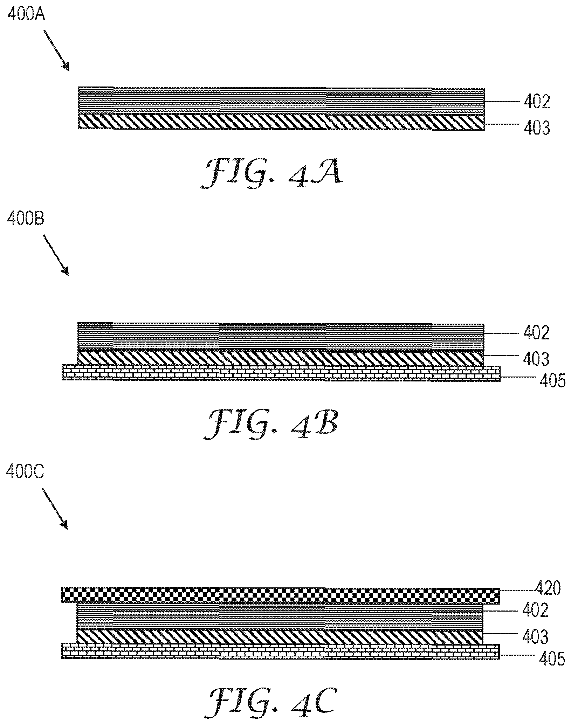

**FIG. 4A** depicts a cross-sectional view of a trapezoidal surgical incise drape **400A** described herein. Surgical incise drape **400A** is shown having a flexible substrate **402** and an antimicrobial adhesive composition **403** in contact with flexible substrate **402.**

**FIG. 4B** depicts a cross-sectional view of a trapezoidal surgical incise drape **400B** described herein. Surgical incise drape **400B** is shown having a flexible substrate **402** and an antimicrobial adhesive composition **403** in contact with flexible substrate **402,** and a release liner **305** in contact with antimicrobial adhesive composition **403.**

**FIG. 4C** depicts a cross-sectional view of a trapezoidal surgical incise drape **400C** described herein. Surgical incise drape **400C** is shown having a flexible substrate **402** and an antimicrobial adhesive composition **403** in contact with flexible substrate **402,** a release liner **405** in contact with antimicrobial adhesive composition **403,** and a delivery system **420** in contact with flexible substrate **402.**

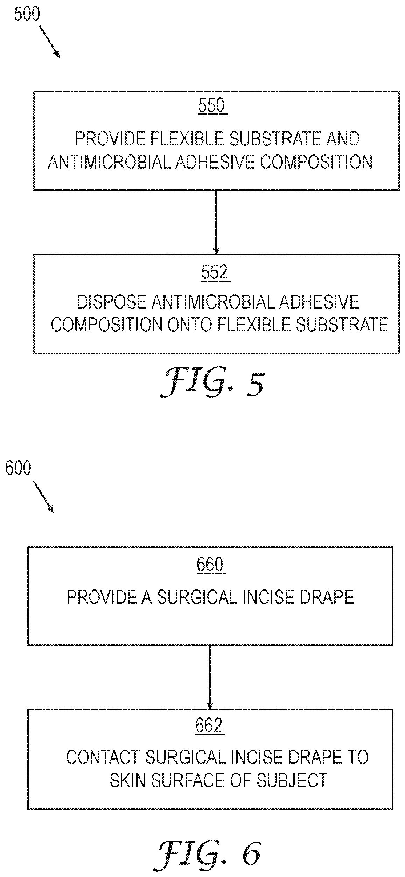

**FIG. 5** is a flow chart representing a method **500** for preparing a surgical incise drape of the present disclosure. The method includes **550** providing a flexible substrate and an antimicrobial adhesive composition and **552** disposing the antimicrobial adhesive composition onto a major surface of the flexible substrate.

**FIG. 6** is a flow chart representing a method **600** for preparing a shoulder for surgery. The method includes **660** providing a surgical incise drape of the present disclosure and **662** contacting the surgical incise drape to a skin surface of a subject.

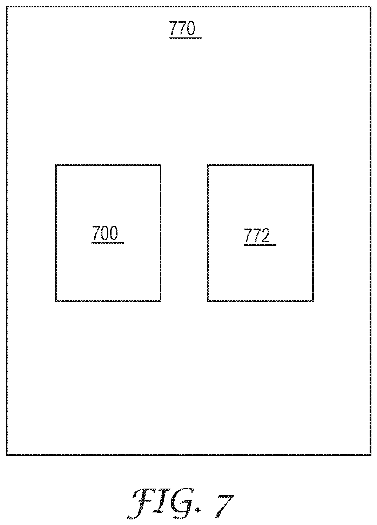

**FIG. 7** depicts a kit **770** including one or more surgical incise drape **700** of the present disclosure and a set of instructions **772** directing a user to contact surgical incise drape **700** to a skin surface of a subject.

### Surgical Incise Drapes

(*One-piece drape*) In various embodiments, a surgical incise drape is described. The surgical incise drape may include a flexible substrate having a trapezoidal configuration characterized by four vertices A, B, C, and D; and an antimicrobial adhesive composition. The flexible substrate may include a first base (*AB*) defined by a first base length (*AB*_{L}) measured between vertices A and B; a second base (*CD*) defined by a second base length (*CD*_{L}) measured between vertices C and D, wherein *AB*_{L} *> CD*_{L}; a first side *(AC)* defined by a first side length (*AC*_{L}) measured between vertices A and C; and a second side *(BD)* defined by a second side length (*BD*_{L}) measured between vertices B and D. The antimicrobial adhesive composition may include an antimicrobial agent and a pressure-sensitive adhesive.

(*Three-piece drape*) In various embodiments, a surgical incise drape is described. The surgical incise drape may include a flexible substrate divided into three separate sections, wherein the three separate sections together form a trapezoidal configuration characterized by four vertices A, B, C, and D; and an antimicrobial adhesive composition. The flexible substrate may include a first base (*AB*) defined by a first base length (*AB*_{L}) measured between vertices A and B; a second base *(CD)* defined by a second base length (*CD*_{L}) measured between vertices C and D, wherein *AB*_{L} *> CD*_{L}; a first side *(AC)* defined by a first side length (*AC*_{L}) measured between vertices A and C; and a second side *(BD)* defined by a second side length (*BD*_{L}) measured between vertices B and D. The antimicrobial adhesive composition may include an antimicrobial agent and a pressure-sensitive adhesive.

In some embodiments, any surgical incise drape described herein may further include a delivery system described herein in contact with at least a portion of the flexible substrate. Variations of delivery system features are described in further detail below.

In some embodiments, any surgical incise drape described herein may further include a release liner described herein in contact with the antimicrobial adhesive composition. Variations of release liner features are described in further detail below.

In some embodiments, any surgical incise drape described herein may further include printed markings. The printed markings may serve as a cutting guide for users to customize the surgical incise drape. Printed markings may also serve as visual placement guides that help a user appropriately apply the drape to the intended area. Customizing the surgical incise drape may allow for a better fit or easier application. In some embodiments, the printed markings extend from the first side to the second side. The printed markings may serve to divide a one-piece surgical incise drape described herein into a three-piece surgical incise drape described herein.

### Flexible Substrates

In some embodiments, the flexible substrate may include a polyurethane, a polyester, a polyether block amide, a thermoplastic polyester, or a combination thereof. In some embodiments, the flexible substrate is a film, e.g., a film formed from one or more of the aforementioned polymers. In some embodiments, the flexible substrate may be transparent or translucent.

In some embodiments, the flexible substrate is characterized by maximum length (i.e., greatest distance measured between *AB* and *CD*) of about 0.5 m to about 0.7 m. For example, the maximum length may be (in meters) of about 0.5, 0.55, 0.6, 0.65, and 0.7, or a value between any of the preceding numbers, e.g., between about 0.55 and about 0.65, between about 0.6 and about 0.7, or the like.

In some embodiments, the flexible substrate may have a thickness of about 25 µm to about 200 µm. For example, the flexible substrate may have a thickness in µm of about 25, 30, 35, 40, 45, 50, 55, 60, 65, 70, 75, 80, 85, 90, 95, 100, 110, 120, 130, 140, 150, 160, 170, 180, 190, and 200, or a value within a range between any of the preceding values, e.g., between about 50 µm and about 80 µm, between about 30 µm and about 120, or the like.

In some embodiments, first base *AB* and second base *CD* are parallel with one another.

In some embodiments, the dimensions may be selected based on the arm structure size of an average human male, female, or child.

In some embodiments, the dimensions may be customized according to the arm structure size of the subject.

In some embodiments, the flexible substrate is in a one-piece configuration having three regions: a drape seal section, an incise site seal section, and a stockinette seal section. In other embodiments, the flexible substrate is in a three-piece configuration, i.e., the surgical incise drape includes three separated flexible substrate subsections, i.e., a drape seal section, an incise site seal section, and a stockinette seal section. Details of the substrate subsection are further described below.

In some embodiments, the flexible substrate may further include contouring along one or more of first base *AB,* second base *CD,* first side *AC,* and second side *BD.* Contouring makes up no greater than about ± 15% of the total surface are of the trapezoid. In other words, the overall area removed and/or gained by contouring can be no greater than about 15% of the trapezoid surface area defined by the area between vertices A, B, C, and D. For example, contouring may add or subtract an area of about 15%, 14%, 13%, 12%, 11%, 10%, 9%, 8%, 7%, 6%, 5%, 4%, 3%, 2%, or about 1% of the overall flexible substrate surface area. Contouring is described in more detail below.

### Characteristics of flexible substrate subsections

The one-piece surgical incise drape construction and the three-piece surgical incise drape construction are designed to overlay the subject's arm extending from the neck to the wrist. The three subsections include: the drape seal, the incise site seal, and the stockinette seal.

In some embodiments, the drape seal subsection may extend from first base *AB* to about 20% to about 33% of the maximum length of the flexible substrate. For example, the drape seal subsection may extend from first base *AB* to about 20%, 21%, 22%, 23%, 24%, 25%, 26%, 27%, 28%, 29%, 30%, 31%, 32%, or 33% of the maximum length of the flexible substrate, or a value between any of the preceding numbers, e.g., between 20% and about 25%, between about 25% and about 30%, or the like.

In some embodiments, the drape seal subsection may have a length of about 0.07 m to about 0.15 m. For example, the drape seal subsection may have a length in m of about 0.07, 0.08, 0.09, 0.10, 0.11, 0.12, 0.13, 0.14, or 0.15, or a value between any of the preceding numbers, e.g., between about 0.9 and about 0.12, between about 0.10 and about 0.14, or the like.

In some embodiments, the drape seal subsection in a three-piece construction may include contouring along one or more of first base *AB* and the base opposite first base *AB.* In other embodiments, the drape seal subsection may include printed markings and/or perforations for cutting such contourings.

In some embodiments, the drape seal subsection has concave contouring along first base *AB.* In some embodiments, the contouring may be semicircular or semielliptical in shape with a radius measurement of about 0.07 m to about 0.3 m. See, for example, **FIG. 1F** **(126).** For example, the contouring may have a radius measurement in m of about 0.07, 0.08, 0.09, 0.10, 0.12, 0.14, 0.16, 0.18, 0.20, 0.22, 0.24, 0.26, 0.28, or 0.30, or a value between any of the preceding numbers, e.g., between about 0.10 and about 0.20, between about 0.14 and about 0.24, or the like.

In some embodiments, the drape seal subsection has convex contouring along the base opposite first base *AB.* In some embodiments, the contouring may be semicircular or semielliptical in shape with a radius measurement of about 0.07 m to about 0.15 m. See, for example, **FIG. 2E**

**(234).** For example, the contouring may have a radius measurement in m of about 0.07, 0.08, 0.09, 0.10, 0.11, 0.12, 0.13, 0.14, or 0.15, or a value between any of the preceding numbers, e.g., between about 0.08 and about 0.12, between about 0.13 and about 0.15, or the like.

In some embodiments, the surgical incise seal subsection may extend from about 33% to about 50% of the maximum length of the flexible substrate. For example, the surgical incise seal subsection may extend from about 33%, 34%, 35%, 36%, 37%, 38%, 39%, 40%, 41%, 42%, 43%, 44%, 45%, 46%, 47%, 48%, 49%, or 50% of the maximum length of the flexible substrate, or a value between any of the preceding numbers, e.g., between about 35% to about 40%, between about 40% and about 45%, or the like.

In some embodiments, the surgical incise seal subsection may have a length of about 0.2 m to about 0.3 m. For example, the surgical incise seal subsection may have a length in m of about 0.20, 0.21, 0.22, 0.23, 0.24, 0.25, 0.26, 0.27, 0.28, 0.29, or 0.30, or a value between any of the preceding numbers, e.g., between about 0.20 and about 0.25, between about 0.25 and about 0.28, or the like.

In some embodiments, the surgical incise seal subsection in a three-piece construction may include contouring along a surface proximate the drape seal subsection and/or a surface proximate the stockinette seal subsection. In other embodiments, the surgical incise seal subsection may include printed markings and/or perforations for cutting such contourings.

In some embodiments, the surgical incise seal subsection in a three-piece construction may include convex contouring along a surface proximate the drape seal subsection. In some embodiments, the contouring may be semicircular or semielliptical in shape with a radius measurement of about 0.07 m to about 0.15 m. See, for example, **FIG. 2E** **(236).** For example, the contouring may have a radius measurement in m of about 0.07, 0.08, 0.09, 0.10, 0.11, 0.12, 0.13, 0.14, or 0.15, or a value between any of the preceding numbers, e.g., between about 0.08 and about 0.12, between about 0.13 and about 0.15, or the like.

In some embodiments, the stockinette seal subsection may extend from second base *CD* to about 25% to about 33% of the maximum length of the flexible substrate. For example, the stockinette seal subsection may extend from the second base *CD* to about 25%, 26%, 27%, 28%, 29%, 30%, 31%, 32%, or 33%, or a value between any of the preceding numbers, e.g., between about 25% and about 30%, between about 28% and about 32%, or the like.

In some embodiments, the stockinette seal subsection may have a length of about 0.15 m to about 0.25 m. For example, the stockinette seal subsection may have a length in m of about 0.15, 0.16, 0.17, 0.18, 0.19, 0.20, 0.21, 0.22, 0.23, 0.24, or 0.25, or a value between any of the preceding numbers, e.g., between about 0.15 and about 0.20, between about 0.20 and about 0.25, or the like.

In some embodiments, the stockinette seal subsection in a three-piece construction may include contouring along one or more of second base *CD* and the base opposite first base *CD.* In other embodiments, the stockinette seal subsection may include printed markings and/or perforations for cutting such contourings.

In some embodiments, the stockinette seal subsection in a three-piece construction may include convex contouring along second base *CD.* In some embodiments, the contouring may be semicircular or semielliptical in shape with a radius measurement of about 0.07 m to about 0.15 m. See, for example, **FIG. 2D** **(230).** For example, the contouring may have a radius measurement in m of about 0.07, 0.08, 0.09, 0.10, 0.11, 0.12, 0.13, 0.14, or 0.15, or a value between any of the preceding numbers, e.g., between about 0.08 and about 0.12, between about 0.13 and about 0.15, or the like.

### Characteristics of Bases AB and CD

In some embodiments, first base *AB* and second base *CD* may have *a AB*_{L}: *CD*_{L} ratio of at least about 2:1. For example, the *AB*_{L}:*CD*_{L} ratio may be about 2:1 to about 7:1. For example, the *AB*_{L}:*CD*_{L} ratio may be selected from about 2:1, 2.5:1, 3:1, 3.5:1, 4:1, 4.5:1, 5:1, 5.5:1, 6:1, 6.5:1, and 7:1, or a value within a range between any of the preceding values, e.g., between about 2:1 and about 4:1, between about 3:1 and about 5:1, or the like.

In many embodiments, the first base *AB* and second base *CD* dimensions (i.e., first base length *AB*_{L} and second base length *CD*_{L}) may be independently selected in any combination from the values listed below, provided that *AB*_{L} *> CD*_{L}*.*

### First base AB

In other embodiments, first base *AB* may have a curvature with respect to a straight line measured between vertices and A and B. For example, first base *AB* may have a parabolic curvature configured to drape around a neck portion.

In some embodiments, the first base *AB* may have a first base length *AB*_{L} selected from about 0.15 m to about 0.65 m. For example, the first base *AB* may have a first base length *AB*_{L}, in m of about 0.15, 0.20, 0.25, 0.30, 0.35, 0.40, 0.45, 0.50, 0.55, 0.60, and 0.65, or a value within a range between any of the preceding values, e.g., between about 0.20 and about 0.40, between about 0.35 and about 0.60, or the like.

In some embodiments, the first base *AB* may include contouring as described in more detail below.

### Second base CD

In some embodiments, the second base *CD* may have a second base length *CD*_{L} selected from about 0.07 m to about 0.3 m. For example, the second base *CD* may have a second base length *CD*_{L}*,* in m of about 0.07, 0.1, 0.12, 0.14, 0.16, 0.18, 0.2, 0.22, 0.24, 0.26, 0.28, and 0.3, or a value within a range between any of the preceding values, e.g., between about 0.14 and about 0.22, between about 0.12 and about 0.26, or the like.

In some embodiments, the second base *CD* may include contouring as described in more detail below.

### AB / CD Contouring

In some embodiments, one or more of first base *AB* and second base *CD* may include contouring. The contouring may be concave, convex, or a combination thereof.

In some embodiments, first base *AB* may include concave contouring designed to traverse the area over the clavicle and trapezius muscle. In some embodiments, the contouring may be semicircular or semielliptical in shape with a radius measurement of about 0.07 m to about 0.6 m. See, for example, **FIG. 1F****.** The omitted surface area of the concave contouring may be about less than 3% of the total flexible substrate surface area. For example, the contouring may have a radius measurement in m of about 0.07, 0.08, 0.09, 0.10, 0.12, 0.14, 0.16, 0.18, 0.20, 0.22, 0.24, 0.26, 0.28, 0.30, 0.32, 0.34, 0.36, 0.38, 0.40, 0.42, 0.44, 0.46, 0.48, 0.50, 0.52, 0.54, 0.56, 0.58, or 0.60, or a value between any of the preceding numbers, e.g., between about 0.08 and about 0.12, between about 0.13 and about 0.15, or the like.

In some embodiments, second base *CD* may include concave contouring designed to encircle the wrist. The contouring may be semicircular or semielliptical in shape with a radius measurement of about 0.07 m to about 0.15 m. See, for example, **FIG. 2D****.** The omitted surface area of the concave contouring may be about less than 2% of the total flexible substrate surface area. For example, the contouring may have a radius measurement in m of about 0.07, 0.08, 0.09, 0.10, 0.11, 0.12, 0.13, 0.14, or 0.15, or a value between any of the preceding numbers, e.g., between about 0.08 and about 0.12, between about 0.13 and about 0.15, or the like.

### Characteristics of Sides AC and BD

In some embodiments, first side *AC* and second side *BD* may have a *AC*_{L}:*BD*_{L} ratio of about 1:1. In some embodiments, the *AC*_{L}:*CD*_{L} ratio may be about 1:1 to about 3:1. For example, the *AC*_{L}:*BD*_{L} ratio may be selected from about 1:1, 1.1:1, 1:1.2, 1.3:1, 1.4:1, 1.5:1, 2:1, 2.5:1, or a value within a range between any of the preceding values, e.g., between about 1:1 and about 1:1.5, between about 1.5:1 and about 2:1, or the like.

In many embodiments, the first side *AC* and second side *CD* dimensions (i.e., first side length *AC*_{L} and second side length *BD*_{L}) may be independently selected in any combination from the values listed below.

### First Side AC / Second side BD

In some embodiments, first side length *AC*_{L} and second side length *BD*_{L} may be independently selected from about 0.35 m to about 0.75 m. For example, the first side length *AC*_{L} and the second side length *BD*_{L} may independently selected, in m, of about 0.35, 0.4, 0.45, 0.5, 0.55, 0.6, 0.65, 0.7, and 0.75, or a value within a range between any of the preceding values, e.g., between about 0.4 and about 0.6, between about 0.5 and about 0.7, or the like.

### AC / BD Contouring

In some embodiments, one or more of first side *AC* and second side *BD* may include contouring. The contouring may be concave, convex, or a combination thereof.

In some embodiments, one or more of first side *AC* and second side *BD* may include concave contouring designed to allow for the surgical incise drape to conform around the curvature of the shoulder. The contouring may be located at about 20% to about 33% of the side length measured from vertices A and/or B. For example, the contouring may be located at about 0.07 m to about 0.15 m from vertices A and/or B along the respective side. In some embodiments, the contouring may have a depth or local minimum of about 0.01 m to about 0.06 m. For example, the contouring may have depth or local minimum, in m, of about 0.01, 0.015 0.02, 0.025, 0.03, 0.035, 0.04, 0.045, 0.05, 0.055, or 0.06, or a value within a range between any of the preceding numbers, e.g., between about 0.025 and about 0.05, between about 0.03 and about 0.04, or the like. The concave contouring may conceptually separate the region defined as the drape seal and the region defined as the surgical incise seal. See, for example, **FIG. 1F****.**

In some embodiments, one or more of first side *AC* and second side *BD* may include concave contouring designed to allow for the surgical incise drape to conform around the curvature of the elbow. The contouring may be located at about 25% to about 33% of the side length measured from vertices C and/or D. For example, the contouring may be located at about 0.20 m to about 0.25 m from vertices C and/or D along the respective side. In some embodiments, the contouring may have a depth or local minimum of about 0.01 m to about 0.05 m. For example, the contouring may have depth or local minimum, in m, of about 0.01, 0.015 0.02, 0.025, 0.03, 0.035, 0.04, 0.045, or 0.05, or a value within a range between any of the preceding numbers, e.g., between about 0.025 and about 0.05, between about 0.03 and about 0.04, or the like. The concave contouring may conceptually separate the region defined as the surgical incise seal and the region defined as the stockinette seal. See, for example, **FIG. 2D****.**

### Characteristics of Angles A_{θ}, B_{θ}, C_{θ}, and D_{θ}

As used herein, angle A_{θ} is defined as the angle formed between first base *AB* and first side *AC,* angle B_{θ} is defined as the angle formed between first base *AB* and second side *BD,* angle C_{θ} is defined as the angle formed between first side *AC* and second base *CD,* and angle D_{θ} is defined as the angle formed between second base *CD* and second side *BD.*

In some embodiments, A_{θ} = B_{θ}.

In some embodiments, C_{θ} = D_{θ}.

In some embodiments, A_{θ} = B_{θ} and C_{θ} = D_{θ} and A_{θ} ≠ C_{θ}.

In some embodiments, A_{θ} < B_{θ}.

In some embodiments, C_{θ} > D_{θ}.

In some embodiments, A_{θ} < B_{θ} and C_{θ} > D_{θ} and A_{θ} ≠ D_{θ}.

In many embodiments, angles A_{θ}, B_{θ}, C_{θ}, and D_{θ}, and any combination thereof, may be selected from the values described below.

### Angle A_{θ} / Angle B_{θ}

In some embodiments, angles A_{θ} and angle B_{θ} may be independently selected from about 75° to about 89°. For example, angle A_{θ} and angle B_{θ} may be independently selected from about 75°, 76°, 77°, 78°, 79°, 80°, 81°, 82°, 83°, 84°, 85°, 86°, 87°, 88°, and 89°, or a value within a range between any of the preceding values, e.g., between about 80° to about 85°, between about 77° and about 87°, or the like.

### Angle C_{θ} / Angle D_{θ}

In some embodiments, angle C_{θ} and angle D_{θ} may be independently selected from about 100° to about 105°. For example, angle C_{θ} angle D_{θ} may be independently selected from about 100°, 101°, 102°, 103°, 104°, and 105°, or a value within a range between any of the preceding values, e.g., between about 100° to about 102°, between about 101° and about 104°, or the like.

### Antimicrobial Adhesive Compositions

In many embodiments, the antimicrobial adhesive composition may include one or more antimicrobial agent described below and one or more pressure-sensitive adhesive described below.

In some embodiments, the antimicrobial adhesive composition may consist essentially of one or more antimicrobial agent described below and one or more pressure-sensitive adhesive described below.

In some embodiments, the antimicrobial agent is completely dissolved within the pressure-sensitive adhesive. In other embodiments, the antimicrobial agent is at least partly suspended within the pressure-sensitive adhesive. The antimicrobial agent should readily diffuse through the pressure-sensitive adhesive so as to supply the surface of the composition, and therefore the skin surface in contact with the composition, with the antimicrobial agent in order to continually disinfect the skin surface.

In some embodiments, the antimicrobial adhesive composition may further include one or more of a plasticizer, a thickener, a tackifier, a stabilizer, an initiator, a crosslinking agent, a solubilizing agent (e.g., monoacylglyceride), a viscosity modifier, a surfactant (e.g., nonionic, cationic, anionic, zwitterionic surfactant), a moisturizing agent, and a vitamin and/or mineral. In some embodiments, the antimicrobial adhesive composition may further include agents to improve stability, viscosity, adhesion, or the like.

### Antimicrobial agents

Generally, the antimicrobial adhesive composition may include any antimicrobial agent, i.e., a chemical compound or complex, known to a skilled artisan, that has been demonstrated to kill infectious organisms (e.g., *Propionibacterium acnes*)*,* or otherwise prevent or slow the reproduction thereof. Antimicrobial agents may include antiseptics, antibacterials, antibiotics, antivirals, antifungals, antiparasitics, or the like.

In some embodiments, the antimicrobial adhesive composition may include an antimicrobial agent selected from iodine, an iodophor, a triiodide complexe, chlorhexidine gluconate, chlorhexidine acetate, octenidine hydrochloride, octenidine alexidine, polymeric biguanides (e.g., polyhexamethylene biguanide), silver, a silver salt (e.g., silver chloride, silver oxide), a copper salt, a polyquaternium, a phenol (e.g., parachlorometaxylenol (PCMX), triclosan, hexachlorophene), fatty acid monoesters (e.g., glycerol monolaurate), hydrogen peroxide, and a combination thereof.

In some embodiments, the antimicrobial adhesive composition may include an antimicrobial agent selected from iodine, an iodophor, chlorhexidine gluconate, octenidine hydrochloride, polyhexamethylene biguanide, a silver salt, a copper salt, and a combination thereof.

In some embodiments, the antimicrobial agent is iodine or an iodophor (e.g., povidone iodine).

In some embodiments, the antimicrobial agent is chlorhexidine gluconate.

In some embodiments, the antimicrobial agent is octenidine hydrochloride.

In some embodiments, the antimicrobial agent is polyhexamethylene biguanide.

### Pressure-Sensitive Adhesives

Generally, the antimicrobial adhesive composition may include any pressure-sensitive adhesive, known to a skilled artisan, that has been shown to be suitable and effective in adhering to skin.

In some embodiments, the pressure-sensitive adhesive may be selected from acrylates, polyurethanes, polyvinyl ethyl ethers, silicones, rubber-based adhesives (e.g., natural rubber, polyisoprene, polyisobutylene, butyl rubber), a combination thereof, and a copolymer thereof. For example, the pressure-sensitive adhesive may include polymers available from Shell Chemical Company (Houston, TX) under the name KRATON.

In some embodiments, the pressure-sensitive adhesive is selected from an acrylic polymer or copolymer. In some embodiments, the acrylic polymer or copolymer may be the reaction product one of monomers selected from an alkyl (meth)acrylate, N-vinyl pyrrolidone, N-vinyl caprolactam, (alkyl-substituted)acrylamide, (alkyl-substituted)methacrylamide, 2-hydroxyethyl (meth)acrylate or and a combination thereof.

In some embodiments, the pressure-sensitive adhesive may be characterized by a glass transition temperature (T_{g}) of about -70 °C to about 20 °C. In some embodiments, the pressure-sensitive adhesive may be characterized by a T_{g} in °C of about -70, -60, -50, -40, -30, -20, -10, -5, 0, 5, 10, or 20, or a value within a range between any of the preceding values, for example, between about -20 and about 5, between about -50 and about -30, or the like.

### Delivery Systems

A delivery system may provide additional stability to the flexible substrate. Surgical incise drapes having a delivery system may mitigate wrinkling upon applying the surgical incise drape to a skin surface. A delivery system may further assist in placement of the surgical incise drape. The delivery system may be removed in sections to further assist delivery.

The delivery system may be constructed from a material more rigid than that of the flexible substrate. For example, the delivery system may include one or more materials selected from polyethylene/vinyl acetate copolymer, polyvinylacetate, polyester film, polyethylene film, nonwovens, polymer films, and papers. The delivery system may be releasably secured to the flexible substrate with a suitable adhesive.

In some embodiments, the delivery system may overlay at least a portion of the flexible substrate. For example, the delivery system may overlay about 10 % to about 100 % of the surface area of the flexible substrate. For example, the delivery system may overlay (in surface area %) about 10, 15, 20, 25, 30, 35, 40, 45, 50, 55, 60, 65, 70, 75, 80, 85, 90, 100, or a value within a range between any of the preceding values, e.g., between about 15 and about 40, between about 60 and about 80, or the like.

In some embodiments, the delivery system may include a perimeter liner, i.e., a material adhered to the perimeter of the surgical incise drape. Perimeter liners may be comprised of one or more pieces.

In some embodiments, the delivery may include a surface liner, i.e., a material adhered to at least a portion of the surface of the surgical incise drape. Surface liners may be configured as a sheet or may be of a patterned configuration. Surface liners may be comprised of one or more pieces.

In some embodiments, the delivery system may include a perimeter liner and a surface liner. In some embodiments, the material of the perimeter liner and the surface liner may be the same or different.

In some embodiments, the delivery system may include one or more perforations that may assist in sectioned removal of the delivery system. Perforations may be partial cuts or other means of dividing a material into sections.

In some embodiments, the delivery system may include a surface liner extending over more than 80% of the flexible substrate surface. The surface liner may be perforated or otherwise divided into sections. In some embodiments, the surface liner may include three sections. In some embodiments, the surface liner may have one or more perforations extending from the first base to the second base. In other embodiments, the surface liner may have one or more perforations extending from the first side to the second side.

In some embodiments, the delivery may include one or more handling tabs. The handling tabs may extend beyond the perimeter of the flexible substrate and/or above the surface of the flexible substrate. Handling tabs may assist a user in appropriately placing the surgical incise drapes, or sections thereof, to a skin surface.

### Release Liners

The release liner may include or be a kraft paper, a glassine paper, polyethylene, polypropylene, polyester, and a combination thereof.

In some embodiments, the release liner may be perforated. Perforations in the release liner may allow for the application of the surgical incise drape in sections. In some embodiments, the surface liner may have one or more perforations extending from the first base to the second base. In other embodiments, the surface liner may have one or more perforations extending from the first side to the second side.

### Methods of Preparation

In one embodiment, a method of making a trapezoidal surgical incise drape is described. The method may include providing a flexible substrate and an antimicrobial adhesive composition; and disposing the antimicrobial adhesive composition onto one or more of the flexible substrate.

In some embodiments, the method may further include providing a release liner and contacting the release liner to the antimicrobial adhesive composition.

In some embodiments, the method may further include providing a delivery system and adhering the delivery system to a surface of the flexible substrate that is opposite the surface having the antimicrobial adhesive composition disposed thereon.

### Methods of Using

In many embodiment, a method for preparing a shoulder for surgery is described. The method may include providing a trapezoidal surgical incise drape described herein; and contacting the surgical incise drape to a skin surface of a subject.

In some embodiments, the skin surface may include a portion of the neck, the shoulder, and the surfaces therebetween. The skin surface may include the shoulder, the upper arm (e.g.,. bicep and tricep), and the surfaces therebetween. The skin surface may include the forearm, the elbow, the wrist, and the surfaces therebetween The skin surface may include the armpit area, the under the upper arm, and the surfaces therebetween.

In some embodiments, the method may further include providing one or more rectangular surgical incise drapes and contacting the one or more rectangular surgical incise drapes to the skin surface and to a portion of the trapezoidal surgical incise drape. Placement of the rectangular surgical incise drape secures the trapezoidal surgical incise drape to the skin surface. The rectangular incise drape may only differ from the trapezoidal surgical incise drapes described herein by shape. The rectangular incise drapes may be wrapped around one or more portions of any arm structure. Rectangular surgical incise drapes may be cut to any desired size.

In some embodiments, a surgical incise drape is first applied to the axilla region. Next, a second surgical incise drape, or the drape seal subsection thereof, is attached to the top of the subject's shoulder. Following afterward, the surgical incise subsection is applied to the subject's upper arm area and then the forearm and wrist. In some embodiments, a middle portion of a release liner is then removed upon anchoring the drape to the skin, following by removal of left and right release liner portions prior to wrapping the drape around the arm.

### Kits

In various embodiments, a kit is described. The kit may include one or more surgical incise drapes described herein and a set of instructions directed a user to perform method steps for preparing a shoulder for surgery described herein.

In some embodiments, the kit may include a one-piece surgical incise drape described herein.

In some embodiments, the kit may include a three-piece surgical incise drape described herein.

In some embodiments, the kit may further include one or more rectangular surgical incise drapes described herein.

In some embodiments, the set of instructions may further direct a user in how to optimally apply the trapezoidal surgical incise drape to the skin surface.

## Claims

1. A surgical incise drape (100A, 100B, 100C, 100D, 100E, 100F, 200A, 200B, 200C, 200D, 200E, 300, 400A, 400B, 400C) comprising:
a flexible substrate (102, 202, 402); and
an antimicrobial adhesive composition (403) comprising:
an antimicrobial agent, and
a pressure-sensitive adhesive;
**characterized in that**:
the flexible substrate (102, 202, 402) has a trapezoidal configuration including four vertices A, B, C, and D; and **in that**
the flexible substrate (102, 402) comprises:
a first base *AB* defined by a first base length *AB*_{L} measured between vertices A and B,
a second base *CD* defined by a second base length *CD*_{L} measured between
vertices C and D,
wherein *AB*_{L} *> CD*_{L}*,*
a first side *AC* defined by a first side length *AC*_{L} measured between vertices A and C, and
a second side *BD* defined by a second side length *BD*_{L} measured between vertices B and D.

2. The
surgical incise drape (200A, 200B, 200C, 200D, 200E, 300, 400A, 400B, 400C of claim 1,
wherein the flexible substrate (202. 402) is divided into three separate subsections, and
wherein the three separate subsections together form the trapezoidal configuration.

3. The surgical incise drape (100A, 100B, 100C, 100D, 100E, 100F, 200A, 200B, 200C, 200D, 200E, 300, 400A, 400B, 400C) of any one of the preceding claims, the flexible substrate characterized having a length from about 0.5 m to about 0.7 m.

4. The surgical incise drape (100A, 100B, 100C, 100D, 100E, 100F, 200A, 200B, 200C, 200D, 200E, 300, 400A, 400B, 400C) of any one of the preceding claims, wherein the ratio *AB*_{L}:*CD*_{L} is at least about 2:1.

5. The surgical incise drape (100A, 100B, 100C, 100D, 100E, 100F, 200A, 200B, 200C, 200D, 200E, 300, 400A, 400B, 400C) of any one of the preceding claims, wherein *AB*_{L} is selected from about 0.15 m to about 0.65 m.

6. The surgical incise drape (100A, 100B, 100C, 100D, 100E, 100F, 200A, 200B, 200C, 200D, 200E, 300, 400A, 400B, 400C) of any one of the preceding claims, wherein *CD*_{L} is selected from about 0.07 m to about 0.3 m.

7. The surgical incise drape (100A, 100B, 100C, 100D, 100E, 100F, 200A, 200B, 200C, 200D, 200E, 300, 400A, 400B, 400C) of any one of the preceding claims, wherein the ratio *AC*_{L}:*BD*_{L} is 1:1.

8. The surgical incise drape (100A, 100B, 100C, 100D, 100E, 100F, 200A, 200B, 200C, 200D, 200E, 300, 400A, 400B, 400C) of any one of the preceding claims, wherein *AC*_{L} and *BD_{L}* are independently selected from about 0.35 m to about 0.75 m.

9. The surgical incise drape (100A, 100B, 100C, 100D, 100E, 100F, 200A, 200B, 200C, 200D, 200E, 300, 400A, 400B, 400C) of any one of the preceding claims, wherein AB and *AC* form an angle A_{θ}, and *AB* and *BD* form an angle B_{θ}, wherein angle A_{θ} and angle B_{θ}, are independently selected from about 75° to about 89°.

10. The surgical incise drape (100A, 100B, 100C, 100D, 100E, 100F, 200A, 200B, 200C, 200D, 200E, 300, 400A, 400B, 400C) of any one of the preceding claims, wherein *AC* and *CD* form an angle C_{θ} and *CD* and *BD* form an angle D_{θ}, wherein angle C_{θ} and angle D_{θ} are selected from about 100° to about 105°.

11. The surgical incise drape (100A, 100B, 100C, 100D, 100E, 100F, 200A, 200B, 200C, 200D, 200E, 300, 400A, 400B, 400C) of any one of the preceding claims, wherein one or more of:
the first base *AB* comprising concave contouring,
the second base *CD* comprising concave contouring,
the first side *AC* comprising concave contouring, and
the second side *BD* comprising concave contouring.

12. The surgical incise drape (100A, 100B, 100C, 100D, 100E, 100F, 200A, 200B, 200C, 200D, 200E, 300, 400A, 400B, 400C) of any one of the preceding claims, wherein one or more of:
the antimicrobial agent is selected from iodine, an iodophor, chlorhexidine gluconate, octenidine hydrochloride, polyhexamethylene biguanide, a silver salt, a copper salt, and a combination thereof, and
the pressure-sensitive adhesive is selected from acrylates, polyurethanes, silicones, rubber-based adhesives, and a combination thereof.

13. The surgical incise drape (100A, 100B, 100C, 100D, 100E, 100F, 200A, 200B, 200C, 200D, 200E, 300, 400A, 400B, 400C) of any one of the preceding claims, further comprising a delivery system (420) in contact with at least a portion of the flexible substrate (402).

14. A method of preparing a surgical incise drape of any one of claims 1-13, the method comprising:
providing the flexible substrate (102, 202, 402) and the antimicrobial adhesive composition (403); and
disposing the antimicrobial adhesive composition (403) onto the flexible substrate.

15. A surgical incise drape (100A, 100B, 100C, 100D, 100E, 100F, 200A, 200B, 200C, 200D, 200E, 300, 400A, 400B, 400C) of any one of claims 1-13, for use in preparing a shoulder for surgery.

## Patentansprüche

1. Ein chirurgisches Abdecktuch (100A, 100B, 100C, 100D, 100E, 100F, 200A, 200B, 200C, 2000, 200E, 300, 400A, 400B, 400C), aufweisend:
ein flexibles Substrat (102, 202, 402); und
eine antimikrobielle Klebstoffzusammensetzung (403), aufweisend:
ein antimikrobielles Mittel und
einen Haftklebstoff;
**dadurch gekennzeichnet, dass**
das flexible Substrat (102, 402) eine trapezförmige Konfiguration, die vier Ecken A, B, C und D einschließt, hat, und dadurch dass
das flexible Substrat (102, 202, 402) aufweist:
eine erste Basis *AB*, die durch eine erste Basislänge *AB*_{L}, gemessen zwischen den Ecken A und B, definiert ist,
eine zweite Basis (*CD*), die durch eine zweite Basislänge *CD*_{L}*,* gemessen zwischen den Ecken C und D, definiert ist,
wobei *AB*_{L} *> CD*_{L}*,*
eine erste Seite (AC), die durch eine erste Seitenlänge *AC*_{L}*,* gemessen zwischen den Ecken A und C, definiert ist, und
eine zweite Seite *BD,* die durch eine zweite Seitenlänge *BD*_{L}, gemessen zwischen den Ecken B und D, definiert ist.

2. Das chirurgische Abdecktuch (200A, 200B, 200C, 2000, 200E, 300, 400A, 400B, 400C) nach Anspruch 1,
wobei das flexible Substrat (202, 402) in drei separate Unterabschnitte unterteilt ist, und
wobei die drei separaten Unterabschnitte zusammen die trapezförmige Konfiguration bilden.

3. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei das flexible Substrat **dadurch gekennzeichnet ist, dass** es eine Länge von etwa 0,5 m bis etwa 0,7 m hat.

4. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei das Verhältnis *AB*_{L}:*CD*_{L} mindestens etwa 2:1 beträgt.

5. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei *AB*_{L} aus etwa 0,15 m bis etwa 0,65 m ausgewählt ist.

6. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei *CD*_{L} aus etwa 0,07 m bis etwa 0,3 m ausgewählt ist.

7. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei das Verhältnis *AC*_{L}:*BD*_{L} 1:1 beträgt.

8. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei *AC*_{L} und *BD*_{L} unabhängig voneinander aus etwa 0,35 m bis etwa 0,75 m ausgewählt sind.

9. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei *AB* und *AC* einen Winkel Ae bilden, und *AB* und *BD* einen Winkel Be bilden, wobei der Winkel A_{θ} und der Winkel Be unabhängig voneinander aus etwa 75° bis etwa 89° ausgewählt sind.

10. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei *AC* und *CD* einen Winkel C_{θ} bilden und *CD* und *BD* einen Winkel De bilden, wobei der Winkel Ce und der Winkel De aus etwa 100° bis etwa 105° ausgewählt sind.

11. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei eines oder mehrere von:
die erste Basis *AB* aufweisend eine konkave Konturierung,
die zweite Basis *CD* aufweisend eine konkaver Konturierung,
die erste Seite *AC* aufweisend eine konkave Konturierung und
die zweite Seite *BD* aufweisend eine konkave Konturierung.

12. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, wobei eines oder mehrere von:
das antimikrobielle Mittel aus Jod, einem Jodophor, Chlorhexidingluconat, Octenidinhydrochlorid, Polyhexamethylenbiguanid, einem Silbersalz, einem Kupfersalz und einer Kombination davon ausgewählt ist, und
der Haftklebstoff aus Acrylaten, Polyurethanen, Silikonen, Klebstoffen auf Kautschukbasis und einer Kombination davon ausgewählt ist.

13. Das chirurgische Abdecktuch (100A, 100B, 100C, 100D, 100E, 100F, 200A, 200B, 200C, 2000, 200E, 300, 400A, 400B, 400C) nach einem der vorstehenden Ansprüche, ferner aufweisend ein Zuführsystem (420), das mit mindestens einem Teil des flexiblen Substrats (402) in Kontakt steht.

14. Ein Verfahren zum Vorbereiten eines chirurgische Abdecktuchs nach einem der Ansprüche 1 bis 13, das Verfahren aufweisend:
Bereitstellen des flexiblen Substrats (102, 202, 402) und der antimikrobiellen Klebstoffzusammensetzung (403); und
Aufbringen der antimikrobiellen Klebstoffzusammensetzung (403) auf das flexible Substrat.

15. Ein chirurgisches Abdecktuch (100A, 100B, 100C, 100D, 100E, 100F, 200A, 200B, 200C, 2000, 200E, 300, 400A, 400B, 400C) nach einem der Ansprüche 1 bis 13 zur Verwendung bei der Vorbereitung einer Schulter für eine Operation.

## Revendications

1. Champ d'incision chirurgical (100A, 100B, 100C, 100D, 100E, 100F, 200A, 200B, 200C, 200D, 200E, 300, 400A, 400B, 400C) comprenant :
un substrat souple (102, 202, 402) ; et
une composition adhésive antimicrobienne (403) comprenant :
un agent antimicrobien, et
un adhésif sensible à la pression ;
**caractérisé en ce que**
le substrat souple (102, 402) a une configuration trapézoïdale comportant quatre coins A, B, C et D, et **en ce que**
le substrat souple (102, 202, 402) comprend :
une première base *AB* définie par une première longueur de base *AB*_{L} mesurée entre les coins A et B,
une seconde base *CD* définie par une seconde longueur de base *CD*_{L} mesurée entre les coins C et D,
où *AB*_{L} > *CD*_{L}*,*
un premier côté *AC* défini par une première longueur de côté *AC*_{L} mesurée entre les coins A et C, et
un second côté (*BD*) défini par une seconde longueur de côté *BD*_{L} mesurée entre les coins B et D.

2. Champ d'incision chirurgical (200A, 200B, 200C, 200D, 200E, 300, 400A, 400B, 400C) selon la revendication 1,
dans lequel le substrat souple (202, 402) est divisé en trois sous-sections distinctes, et
dans lequel les trois sous-sections distinctes forment ensemble la configuration trapézoïdale.

3. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, le substrat souple étant **caractérisé en ce qu'**il a une longueur d'environ 0,5 m à environ 0,7 m.

4. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel le rapport *AB*_{L}:*CD*_{L} est d'au moins environ 2:1.

5. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel *AB*_{L} est choisi entre environ 0,15 m et environ 0,65 m.

6. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel *CD*_{L} est choisi entre environ 0,07 m et environ 0,3 m.

7. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel le rapport *AC*_{L}:*BD*_{L} est de 1:1.

8. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel *AC*_{L} et *BD*_{L} sont indépendamment choisis entre environ 0,35 m et environ 0,75 m.

9. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel *AB* et *AC* forment un angle A_{θ}, et *AB* et *BD* forment un angle Be, dans lequel l'angle Ae et l'angle Be sont indépendamment choisis entre environ 75° et environ 89°.

10. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel *AC* et *CD* forment un angle C_{θ} et *CD* et *BD* forment un angle D_{θ}, dans lequel l'angle Ce et l'angle De sont choisis entre environ 100° et environ 105°.

11. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel un ou plusieurs parmi :
la première base *AB* comprenant un contour concave,
le seconde base *CD* comprenant un contour concave,
le premier côté *AC* comprenant un contour concave, et
le second côté *BD* comprenant un contour concave.

12. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, dans lequel un ou plusieurs parmi :
l'agent antimicrobien est choisi parmi iode, un iodophore, gluconate de chlorhexidine, chlorhydrate d'octénidine, polyhexaméthylène biguanide, un sel d'argent, un sel de cuivre et une combinaison de ceux-ci, et
l'adhésif sensible à la pression est choisi parmi acrylates, polyuréthanes, silicones, adhésifs à base de caoutchouc et une combinaison de ceux-ci.

13. Champ d'incision chirurgical (100A, 100B, 100C, 100D, 100E, 100F, 200A, 200B, 200C, 200D, 200E, 300, 400A, 400B, 400C) selon l'une quelconque des revendications précédentes, comprenant en outre un système de pose (420) en contact avec au moins une partie du substrat souple (402).

14. Procédé de préparation d'un champ d'incision chirurgical selon l'une quelconque des revendications 1 à 13, le procédé comprenant :
la fourniture du substrat souple (102, 202, 402) et de la composition adhésive antimicrobienne (403) ; et
la disposition de la composition adhésive antimicrobienne (403) sur le substrat souple.

15. Champ d'incision chirurgical (100A, 100B, 100C, 100D, 100E, 100F, 200A, 200B, 200C, 200D, 200E, 300, 400A, 400B, 400C) selon l'une quelconque des revendications 1 à 13, à utiliser pour la préparation d'une épaule en vue d'une intervention chirurgical.
